(19) Europäisches Patentamt
European Patent Office
Office européen des brevets

(11) **EP 3 777 802 A1**

(12) **EUROPEAN PATENT APPLICATION**
published in accordance with Art. 153(4) EPC

(43) Date of publication:
**17.02.2021 Bulletin 2021/07**

(21) Application number: **19776641.3**

(22) Date of filing: **27.03.2019**

(51) Int Cl.:
***A61F 13/53*** (2006.01)

(86) International application number:
**PCT/JP2019/013330**

(87) International publication number:
**WO 2019/189445 (03.10.2019 Gazette 2019/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.03.2018 JP 2018062079**

(71) Applicant: **Sumitomo Seika Chemicals Co., Ltd.
Kako-gun, Hyogo 675-0145 (JP)**

(72) Inventors:
• **ITO, Takashi
Himeji-shi, Hyogo 672-8076 (JP)**
• **SAKODA, Miki
Himeji-shi, Hyogo 672-8076 (JP)**
• **MATSUOKA, Mutsumi
Himeji-shi, Hyogo 672-8076 (JP)**

(74) Representative: **Nederlandsch Octrooibureau
P.O. Box 29720
2502 LS The Hague (NL)**

(54) **ABSORBENT ARTICLE**

(57) Disclosed is an absorbent article including an absorbent including water-absorbing resin particles, wherein the water-absorbing resin particles satisfy the following requirements (a), (b), and (c).
(a) A release restoration rate represented by Formula (1) is 5% or more.

$$(k_2-k_1)/k_1\times100\ (\%) \ ... \ (1)$$

(b) A water absorption rate measured by a vortex method is 5 seconds or less.
(c) A retention capacity of physiological saline is 15 to 39 g/g.

**Fig.1**

(a)

*Fig.1*

(b)

19

10

13

17

15

$k_1$

11

*Fig.1*

(c)

10

13

17

15

$k_2$

11

## Description

### Technical Field

The present invention relates to an absorbent article.

### Background Art

An absorbent containing water-absorbing resin particles is used in sanitary goods such as paper diapers and sanitary napkins. A conventional absorbent is generally a mixture of water-absorbing resin particles and fibrous materials such as pulp. In recent years, the trend is toward thinner sanitary goods, and the use of pulp-less absorbent is considered.

### Citation List

### Patent Literature

[Patent Literature 1] JP 2015-154881 A

### Summary of Invention

### Technical Problem

The fibrous material such as pulp in the absorbent has a role of assisting diffusion and permeation of the absorbed liquid, and a permeation rate when the absorbent absorbs the liquid depends on a content of the fibrous material in the absorbent. In a conventional absorbent article, in a case where the content of the fibrous material in the absorbent is low, the absorbed liquid does not sufficiently diffuse and permeate, so that a gel blocking effect of the water-absorbing resin particles is likely to appear. Gel blocking is a phenomenon in which the water-absorbing resin particles that have absorbed water swell to form a gel, and the swollen gel is densely packed to block a passage of the liquid, and as a result, further passing or absorption of the liquid is prevented.

In a pulp-less or thinner sanitary goods, an attempt has been made to suppress a decrease in absorption rate by disposing a layer having liquid diffusibility outside the absorbent (for example, Patent Literature 1). However, the use of such a member increases costs and also causes an increase in overall thickness of the absorbent article due to a thickness of the layer itself. Therefore, there is a need for an absorbent article having an excellent permeation rate without using a non-woven fabric having a liquid diffusing ability.

Generally, the higher the water absorption rate of the water-absorbing resin particles is, the more likely the gel blocking effect is to appear. So far, water-absorbing resin particles having both a sufficiently high water absorption rate and a high liquid permeability (anti-gel blocking property) have not been developed.

An object of the present invention is to provide an absorbent article having favorable water absorption performance and a high permeation rate.

### Solution to Problem

The present invention provides an absorbent article comprising an absorbent comprising water-absorbing resin particles, wherein the water-absorbing resin particles satisfy the following requirements (a), (b), and (c).

(a) A release restoration rate represented by Formula (1) is 5% or more:

$$(k_2\text{-}k_1)/k_1 \times 100\ (\%) \ ... \ (1)$$

wherein in Formula (1), $k_1$ represents a height (mm) of the water-absorbing resin particles after a cylinder with an inner diameter of 25.4 mm, which has a mesh-like bottom onto which 0.2 g of the water-absorbing resin particles are sprinkled, is placed in a container having ion exchange water in an amount of 30 times a mass of the water-absorbing resin particles such that the water-absorbing resin particles absorb the ion exchange water and a load of $1.3 \times 10^3$ Pa is applied to the water-absorbing resin particles for 1 minute after 1 minute from initiation of the water absorption, and $k_2$ represents a height (mm) of the water-absorbing resin particles after 1 minute from release of the load applied when measuring $k_1$.

(b) A water absorption rate measured by a vortex method is 5 seconds or less.

(c) A retention capacity of physiological saline is 15 to 39 g/g.

Since the absorbent article comprises the water-absorbing resin particles having favorable water absorption performance and release restoration rate, the absorbent article has favorable water absorption performance and a high permeation rate.

In the absorbent article, a content of the water-absorbing resin particles in the absorbent may be 50 to 80 g/m$^2$.

It is preferable that the absorbent has a thickness of 1.2 mm or less.

It is preferable that the absorbent article has a thickness of 2.0 mm or less. Since the absorbent comprises the water-absorbing resin particles having excellent water absorption performance and liquid permeability, the absorbent article has sufficiently favorable water absorption performance and a high permeation rate even though a thickness thereof is thin.

It is preferable that the absorbent article has a permeation rate of physiological saline of 15 seconds or less.

**Advantageous Effects of Invention**

According to the present invention, it is possible to provide an absorbent article having favorable water absorption performance and a high permeation rate.

**Brief Description of Drawings**

Fig. 1 is a schematic view illustrating a method of measuring a release restoration rate of water-absorbing resin particles.

Fig. 2 is a schematic cross-sectional view illustrating an aspect of an absorbent article according to the present embodiment.

**Description of Embodiments**

Hereinafter, suitable embodiments of the present invention will be described in detail. However, the present invention is not limited to the following embodiments. All configurations described in the present specification can be optionally combined without departing from the scope of the present invention. For example, numerical value ranges regarding various characteristics can be defined using the upper limit values and the lower limit values of the numerical value ranges described in the present specification, or the numerical values optionally selected from the numerical values described in Examples as the upper limit values and the lower limit values.

Water-absorbing resin particles used in an absorbent article according to the present embodiment satisfy the following requirements (a), (b), and (c).

(a) A release restoration rate represented by Formula (1) is 5% or more.

$$(k_2-k_1)/k_1 \times 100 \ (\%) \ ... \ (1)$$

(b) A water absorption rate measured by a vortex method is 5 seconds or less.

(c) A retention capacity of physiological saline is 15 to 39 g/g.

In Formula (1), $k_1$ represents a height (mm) of the water-absorbing resin particles after a cylinder with an inner diameter of 25.4 mm, which has a mesh-like bottom onto which 0.2 g of the water-absorbing resin particles are sprinkled, is placed in a container having ion exchange water in an amount of 30 times a mass of the water-absorbing resin particles such that the water-absorbing resin particles absorb the ion exchange water and a load of $1.3 \times 10^3$ Pa is applied to the water-absorbing resin particles for 1 minute after 1 minute from initiation of the water absorption, and $k_2$ represents a height (mm) of the water-absorbing resin particles after 1 minute from release of the load applied when measuring $k_1$. An example of a detailed method of measuring the release restoration rate will be described in Examples below.

The release restoration rate of the water-absorbing resin particles is 5% or more, preferably 7% or more, more preferably 9% or more, still more preferably 11% or more, and further still more preferably 13% or more. The release restoration rate may be, for example, 25% or less, 22% or less, or 20% or less.

A water absorption rate of the water-absorbing resin particles may be, for example, 1 second or more, and may be 2 seconds or more. A retention capacity of physiological saline of the water-absorbing resin particles may be 15 to 36 g/g. The retention capacity of physiological saline and the absorption rate of physiological saline are measured by methods described in Examples which will be described later.

The water-absorbing resin particles used in the absorbent article according to the present embodiment can be produced, for example, by a production method described below. The water-absorbing resin particles may comprise, for example, a crosslinked polymer obtained by the polymerization of monomers containing water-soluble ethylenically unsaturated monomers.

Examples of a method for polymerizing the monomers include a reverse-phase suspension polymerization method, an aqueous solution polymerization method, a bulk polymerization method, and a precipitation polymerization method. Among these, from viewpoints of making it easy to secure favorable water absorption characteristics of the obtained water-absorbing resin particles and control of the polymerization reaction, the reverse-phase suspension polymerization method or the aqueous solution polymerization method is preferable.

In order to increase a specific surface area of the obtained water-absorbing resin particles, for example, a method in which a nonionic surfactant or an anionic surfactant with a hydrophilic-lipophilic balance (HLB) of 6 or more is used in the reverse-phase suspension polymerization method, a method in which a pyrolytic foaming agent such as an azo compound is used in the aqueous solution polymerization method, or the like can be adopted. Water-absorbing resin particles obtained by the method in which a nonionic surfactant or an anionic surfactant with an HLB of 6 or more is used in the reverse-phase suspension polymerization method among these methods can be suitably used. The HLB of the surfactant used in the reverse-phase suspension polymerization is more preferably 6 to 14, and still more preferably 8 to 12.

Hereinbelow, a method for polymerizing the water-soluble ethylenically unsaturated monomers will be described by way of an example of the reverse-phase suspension polymerization method.

Hereinafter, "acrylic" and "methacrylic" are collectively referred to as "(meth)acrylic". "Acrylate" and "methacrylate" are also referred to as "(meth)acrylate". Examples of the water-soluble ethylenically unsaturated monomer used in the production of the water-absorbing resin particles include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the water-soluble ethylenically unsaturated monomer contains an amino group, the amino group may be quaternarized. A functional group such as a carboxyl group and an amino group, which is contained in the monomer, can function as a crosslinkable functional group in a post-crosslinking process which will be described later. These water-soluble ethylenically unsaturated monomers may be used alone or in a combination of two or more kinds thereof.

From a viewpoint of industrial availability, the water-soluble ethylenically unsaturated monomers preferably comprise, among these, at least one compound selected from the group consisting of acrylic acid and a salt thereof, methacrylic acid and a salt thereof, acrylamide, methacrylamide, and N,N-dimethyl acrylamide, and more preferably comprise at least one compound selected from the group consisting of acrylic acid and a salt thereof, methacrylic acid and a salt thereof, and acrylamide. The water-soluble ethylenically unsaturated monomers still more preferably comprise at least one compound selected from the group consisting of acrylic acid and a salt thereof, and methacrylic acid and a salt thereof, from a viewpoint of further enhancing the water absorption characteristics.

As the monomer, some of water-soluble monomers other than the above-described water-soluble ethylenically unsaturated monomers may also be used to an extent that exhibition of the effect of the present invention is not inhibited. Such a monomer can be used, for example, by mixing with an aqueous solution containing the water-soluble ethylenically unsaturated monomers. It is preferable that the amount of the water-soluble ethylenically unsaturated monomers to be used is 70% to 100% by mole with the total amount of the monomers. Above all, it is more preferable that acrylic acid and a salt thereof are in the amount of 70% to 100% by mole with the total amount of the monomers.

Usually, the water-soluble ethylenically unsaturated monomers are suitably used in a form of an aqueous solution. A concentration of the water-soluble ethylenically unsaturated monomers in an aqueous solution of the water-soluble ethylenically unsaturated monomers (hereinafter, referred to as an aqueous monomer solution) can usually be any value of 20% by mass or more and a saturated concentration or less, and is preferably 25% to 70% by mass, and more preferably 30% to 55% by mass. Examples of water to be used include tap water, distilled water, and ion exchange water.

In a case where the water-soluble ethylenically unsaturated monomer comprises an acid group, the aqueous monomer solution may be used after neutralizing the acid group with an alkaline neutralizing agent. From viewpoints of increasing the osmotic pressure of the obtained water-absorbing resin particles and further enhancing water absorption characteristics such as a water absorption rate, a degree of neutralization in the water-soluble ethylenically unsaturated monomers by the alkaline neutralizing agent is 10% to 100% by mole, preferably 50% to 90% by mole, and more preferably 60% to 80% by mole of the acidic groups in the water-soluble ethylenically unsaturated monomers. Examples of the alkaline neutralizing agent include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution in order to simplify neutralizing operation. The above-mentioned alkaline neutralizing agents may be used alone or in combination of two or more kinds thereof. Neutralization of the acid groups of the water-soluble ethylenically unsaturated monomers can be performed by, for example, adding an aqueous solution of sodium hydroxide, potassium hydroxide, or the like dropwise to the aqueous monomer solution and mixing them.

In the reverse-phase suspension polymerization method, an aqueous monomer solution is dispersed in a hydrocarbon dispersion medium in the presence of a surfactant, and polymerization of the water-soluble ethylenically unsaturated monomers is performed using a water-soluble radical polymerization initiator or the like. An internal crosslinking agent may be used in the polymerization.

Examples of the surfactant include nonionic surfactants and anionic surfactants. Examples of the nonionic surfactants include sorbitan fatty acid esters, (poly)glycerin fatty acid esters ("(poly)" means both of a case with the prefix "poly" and a case without the prefix "poly", and the same applies hereinbelow.), sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymer, polyoxyethylene polyoxypropyl alkyl ethers, and polyethylene glycol fatty acid esters. Examples of the anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Among these, from viewpoints of a favorable state of a W/O type reverse-phase suspension, facilitating the obtaining of water-absorbing resin particles with suitable particle diameters, and industrial availability, the surfactant preferably comprises at least one compound selected from the group consisting of sorbitan fatty acid esters, polyglycerin fatty acid esters, and sucrose fatty acid esters. Furthermore, from a viewpoint of improving the water absorption characteristics of the obtained water-absorbing resin particles, the surfactant more preferably comprises sorbitan fatty acid esters. These surfactants may be used alone or in combination of two or more kinds thereof.

From a viewpoint of obtaining sufficient effect on the amount of the surfactant to be used and from an economical viewpoint, the amount of the surfactant is preferably 0.1 to 5 parts by mass, more preferably 0.2 to 3 parts by mass, and still more preferably 0.5 to 2 parts by mass, with respect to 100 parts by mass of the aqueous solution of the water-soluble ethylenically unsaturated monomers.

Examples of the water-soluble radical polymerization initiator include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-methylpropionamidine) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis {2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). These radical polymerization initiators may be used alone or in combination of two or more kinds thereof. The term "water-soluble" of the water-soluble radical polymerization initiator in the present specification means that the radical polymerization initiator exhibits a solubility of 5% by mass or more in water at 25°C.

The amount of the water-soluble radical polymerization initiator to be used may be 0.005 to 1 mole with respect to 100 moles of the water-soluble ethylenically unsaturated monomers. In a case where the amount of the radical polymerization initiator to be used is 0.005 moles or more, the polymerization reaction does not require a long period of time, which is thus efficient. In a case where the amount of the radical polymerization initiator to be used is 1 mole or less, it is effective in preventing an abrupt polymerization reaction.

The water-soluble radical polymerization initiator can also be used as a redox polymerization initiator in combination with a reducing agent such as sodium sulfite, sodium hydrogen sulfite, ferrous sulfate, and L-ascorbic acid.

In order to control the water absorption characteristics of the water-absorbing resin particles in the polymerization reaction, a chain transfer agent may be contained in the aqueous solution of the water-soluble ethylenically unsaturated monomers used for polymerization. Examples of the chain transfer agent include hypophosphites, thiols, thiolic acids, secondary alcohols, and amines.

Examples of the hydrocarbon dispersion medium include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. The hydrocarbon dispersion medium may comprise at least one compound selected from the group consisting of a chained aliphatic hydrocarbon having 6 to 8 carbon atoms and an alicyclic hydrocarbon having 6 to 8 carbon atoms.

From viewpoints of a favorable state of a W/O type reverse-phase suspension, facilitating the obtaining of water-absorbing resin particles with high water absorption rates and suitable particle diameters, industrial availability, and stable quality, the hydrocarbon dispersion medium may comprise n-heptane, cyclohexane, or the both. In addition, from the same viewpoints, as a mixture of the hydrocarbon dispersion media, for example, Exxsol Heptane (manufactured by ExxonMobil Chemical: containing 75% to 85% of hydrocarbons of n-heptane and isomers thereof), which is commercially available, may be used.

From viewpoint of appropriately removing heat of polymerization and making it easy to control a polymerization temperature, the amount of the hydrocarbon dispersion medium to be used is preferably 100 to 1,000 parts by mass, more preferably 150 to 800 parts by mass, and still more preferably 200 to 700 parts by mass, with respect to 100 parts by mass of the water-soluble ethylenically unsaturated monomers. When the amount of the hydrocarbon dispersion medium to be used is 100 parts by mass or more, there is a tendency that it becomes easy to control the polymerization temperature. When the amount of the hydrocarbon dispersion medium to be used is 1,000 parts by mass or less, there is a tendency that the productivity of polymerization is improved, which is economical.

Internal crosslinking occurs by self-crosslinking upon the polymerization, but the water absorption characteristics of the water-absorbing resin particles may be controlled by carrying out internal crosslinking using further using an internal crosslinking agent. Examples of the internal crosslinking agent to be used include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallylisocyanate, and divinylbenzene; polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. These crosslinking agents may be used alone or in combination of two or more kinds thereof.

From viewpoints of suppressing the properties of water solubility by appropriately crosslinking the obtained polymer and exhibiting sufficient water absorption amounts, the amount of the internal crosslinking agent is preferably 0 to 0.03 moles, more preferably 0 to 0.01 moles, and still more preferably 0 to 0.005 moles, per 1 mole of the water-soluble ethylenically unsaturated monomer.

The reverse-phase suspension polymerization is performed in a water-in-oil system by mixing water-soluble ethylenically unsaturated monomers, a water-soluble radical polymerization initiator, and as desired, an internal crosslinking agent, a surfactant, and a hydrocarbon dispersion medium with each other, and heating the mixture under stirring. The addition procedure of the respective components, and the like can be appropriately adjusted, but it is suitable that, for example, a surfactant and a hydrocarbon dispersion medium are mixed with each other in advance, a water-soluble radical polymerization initiator, an internal crosslinking agent, and water-soluble ethylenically unsaturated monomers are mixed with each other in advance, and mixed liquids thus obtained are mixed with each other, whereby polymerization is initiated. In addition, a multi-stage polymerization method in which the monomer is added multiple times may be used.

A temperature for the polymerization reaction varies depending upon a water-soluble radical polymerization initiator to be used, but from viewpoints that the reaction is smoothly performed by easily removing heat of polymerization while making the polymerization rapidly proceed and shortening the polymerization time to increase the economy, the temperature is preferably 20°C to 110°C, and more preferably 40°C to 90°C. The reaction time is usually 0.5 to 4 hours. Completion of the polymerization reaction can be confirmed by, for example, stopping a rise in the temperature within the reaction system. Thus, the water-absorbing resin particles are usually obtained in the state of a hydrous gel.

After the polymerization reaction, the obtained hydrous gel may be subjected to intermediate crosslinking. By performing the intermediate crosslinking, the degree of crosslinking of the hydrous gel can be increased to improve the water absorption characteristics more preferably. The intermediate crosslinking can be carried out by adding a crosslinking agent to the hydrous gel after the polymerization reaction, followed by heating.

Examples of the crosslinking agent for performing the intermediate crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epi-bromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these, the polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. These crosslinking agents may be used alone or in combination of two or more kinds thereof.

From the viewpoints of suppressing the properties of water solubility by appropriately crosslinking the obtained hydrous gel and exhibiting favorable water absorption characteristics, the amount of the crosslinking agent used for the intermediate crosslinking is preferably 0 to 0.03 moles, more preferably 0 to 0.01 moles, and still more preferably 0 to 0.005 moles, per 1 mole of the water-soluble ethylenically unsaturated monomer.

Subsequently, in order to remove moisture from the obtained hydrous gel, drying is performed. Examples of the drying method include (a) method in which azeotropic distillation is performed by heating from the outside in the state where the hydrous gel is dispersed in a hydrocarbon dispersion medium, and the hydrocarbon dispersion medium is refluxed to remove moisture, (b) method in which a polymer in the form of a hydrous gel is taken out by decantation and dried under reduced pressure, and (c) method in which a polymer in the form of a hydrous gel is separated by filtration with a filter and dried under reduced pressure. Among these, (a) method is preferably used due to simplicity in a production process.

Control over the particle diameter of the water-absorbing resin particle can be performed by, for example, adjusting the rotational speed of a stirrer during the polymerization reaction or by adding a powdery inorganic flocculating agent to the system after the polymerization reaction or at an initial time of drying. The particle diameter of the obtained water-absorbing resin particle can be increased by the addition of the flocculating agent. Examples of the powdery inorganic flocculating agent include silica, zeolite, bentonite, aluminum oxide, talc, titanium dioxide, kaolin, clay, and hydrotalcite, and among these, from a viewpoint of the aggregation effect, silica, aluminum oxide, talc, or kaolin is preferable.

In the reverse-phase suspension polymerization, as a method of adding the powdery inorganic flocculating agent, a method in which a powdery inorganic flocculating agent is dispersed in the same kind of a hydrocarbon dispersion medium as that for use in the polymerization or water in advance, followed by performing mixing in a hydrocarbon dispersion medium containing a hydrous gel under stirring, is preferable.

The amount of the powdery inorganic flocculating agent to be added is preferably 0.001 to 1 part by mass, more preferably 0.005 to 0.5 parts by mass, and still more preferably 0.01 to 0.2 parts by mass, with respect to 100 parts by mass of the water-soluble ethylenically unsaturated monomers. By adjusting the amount of the powdery inorganic flocculating agent to be added to be within the range, it is easy to obtain water-absorbing resin particles having a desired particle size distribution.

The above-mentioned initial time in drying indicates, for example, a state where a water content of the hydrous gel is 50% by mass or more in a drying process after the polymerization reaction. As a specific time point for adding the powdery inorganic flocculating agent according to the present embodiment, a time point at which the water content of the hydrous gel is 50% by mass or more is preferable, a time point at which the water content of the hydrous gel is 55% by mass or more is more preferable, and a time point at which the water content of the hydrous gel is 60% by mass or more is still more preferable.

The water content of the hydrous gel is calculated by the following formula.

$$\text{Water content} = (Ww) \div (Ww + Ws) \times 100 \ [\% \text{ by mass}]$$

Ww: The amount of moisture of a hydrous gel obtained by adding an amount of moisture used, as desired, upon mixing a powdery inorganic flocculating agent, a post-crosslinking agent, and the like to the amount obtained by subtracting the amount of moisture extracted to the outside by the drying process from the amount of moisture contained in the aqueous liquid before polymerization in an entire polymerization process.

Ws: A solid fraction amount calculated from the amount of materials such as water-soluble ethylenically unsaturated monomers, a crosslinking agent, and an initiator, each of which constitutes the hydrous gel polymer, to be introduced.

In the production of the water-absorbing resin particles, it is preferable that a surface portion of the hydrous gel is crosslinked (post-crosslinked) using a crosslinking agent in the drying process or any of the following processes. It is preferable that post-crosslinking is performed at the timing when the hydrous gel has a specific water content. The time of the post-crosslinking is preferably a time point at which the water content of the hydrous gel is 10% to 60% by mass, more preferably a time point at which the water content of the hydrous gel is 20% to 55% by mass, and still preferably a time point at which the water content of the hydrous gel is 30% to 50% by mass.

Examples of the post-crosslinking agent for performing post-crosslinking include compounds having two or more reactive functional groups. Examples of the post-crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among these, the polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These post-crosslinking agents may be used alone or in combination of two or more kinds thereof.

The amount of the post-crosslinking agent cannot be absolutely determined since it varies depending on the type of the post-crosslinking agent, but the amount is usually a ratio of 0.00001 to 0.02 moles, preferably 0.0001 to 0.01 moles, and more preferably 0.0005 to 0.005 moles, in terms of a ratio with respect to 1 mole of the water-soluble ethylenically unsaturated monomers used for polymerization.

From a viewpoint of sufficiently increasing the crosslinking density in the surface portion of the water-absorbing resin particles to enhance the gel strength of the water-absorbing resin particles, the amount of the post-crosslinking agent to be used is preferably 0.00001 moles or more, and from a viewpoint of increasing the water retention capacity of the water-absorbing resin particles, the amount of the post-crosslinking agent to be used is preferably 0.02 moles or less.

In the production of the water-absorbing resin particles, a polyvalent metal compound may be used as a crosslinking agent, for example. The water-absorbing resin particles according to the embodiment may not contain the polyvalent metal compound.

A dried product of the surface-crosslinked water-absorbing resin particles can be obtained by distilling off water and the hydrocarbon dispersion medium by a known method after the post-crosslinking reaction. Water may be added to the particles during the drying. Water can be added, for example by spraying.

The water-absorbing resin particles can be used in mixture with, for example, a gel stabilizer, a metal chelating agent, silica, or the like. The water-absorbing resin particles can be made to have a desired particle size distribution at the time point obtained by the above-mentioned production method, but the particle size distribution may be set to a predetermined particle size distribution by further performing operations such as adjustment of a particle size through classification with a sieve.

In the water-absorbing resin particles, for example, a proportion of the particles having a particle diameter of more than 250 $\mu$m and 850 $\mu$m or less may be 70% by mass or more, 75% by mass or more, 80% by mass or more, 85% by mass or more, or 90% by mass or more, with respect to the total amount of the water-absorbing resin particles. In the water-absorbing resin particles, a proportion of the particles having a particle diameter of 250 $\mu$m or less may be 20% by mass or less, 18% by mass or less, 15% by mass or less, or 10% or less, with respect to the total amount of the water-absorbing resin particles.

The water-absorbing resin particles may have a median particle diameter of, for example, 250 to 850 $\mu$m, and the median particle diameter is preferably 300 to 700 $\mu$m, and more preferably 300 to 500 $\mu$m.

The water-absorbing resin particles generally take shapes such as a substantial spherical shape, a crushed shape, a granular shape, and aggregates thereof, depending on a production method thereof. From a viewpoint of more making it easier to obtain the effect of the invention of the present application, the water-absorbing resin particles according to the present embodiment may be, for example, in the granular shape. The granular shape in the present specification indicates that particles have a number of projections on surfaces thereof, and can also be mentioned as a fine uneven shape. The water-absorbing resin particles according to the present embodiment may also be aggregates of granular particles.

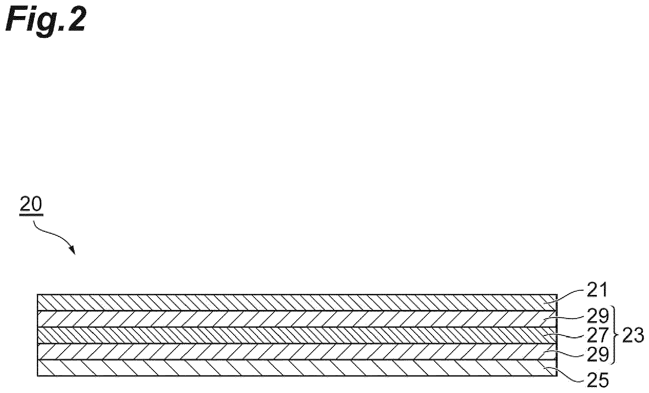

The absorbent article according to the present embodiment comprises an absorbent comprising water-absorbing resin particles. Fig. 2 is a schematic cross-sectional view illustrating an aspect of the absorbent article according to the present embodiment. An absorbent article 20 comprises a liquid-permeable sheet 21, an absorbent 23, and a liquid-impermeable sheet 25 in this order. The absorbent 23 comprises an absorbent core 27 cimprising water-absorbing resin particles, and core wraps 29 for retaining the absorbent core 27. The core wraps 29 are disposed above and below the absorbent core 27 to hold the absorbent core 27 therebetween. The absorbent 23 may be formed of only the absorbent core 27. The core wraps 29 may be, for example, a tissue, a non-woven fabric, or the like. The core wraps 29 may be integrated without being separated, or may have a form that wraps the entire absorbent core 27.

The absorbent core may further comprise a fibrous material. In a case where the absorbent core comprises a fibrous material, the absorbent core may have, for example, a form in which the water-absorbing resin particles and the fibrous material are uniformly mixed.

Examples of the fibrous material include cellulose-based fibers such as finely pulverized wood pulp, cotton, cotton linter, rayon, and cellulose acetate, and synthetic fibers such as polyamides, polyesters, and polyolefins. In addition, the fibrous materials may be a mixture of the above-mentioned fibers.

Fibers may adhere to each other by adding an adhesive binder to the fibrous material in order to enhance the shape retention properties before or during use of the absorbents. Examples of the adhesive binder include hot-melt synthetic fibers, hot-melt adhesives, and adhesive emulsions.

Examples of the hot-melt synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, the polyethylene portion alone is hot-melted. Examples of the hot-melt adhesive include a blend of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a tackifier, a plasticizer, an antioxidant, or the like.

Examples of the adhesive emulsions include polymers of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone or in combination of two or more kinds thereof.

In the absorbent core, a proportion of the water-absorbing resin particles with respect to the total amount of the water-absorbing resin particles and the fibrous materials may be, for example, 2% by mass or more, 5% by mass or more, 10% by mass or more, 20% by mass or more, 30% by mass or more, 50% by mass or more, 70% by mass or more, 80% by mass or more, 90% by mass or more, 95% by mass or more, or 98% by mass or more. The proportion of the water-absorbing resin particles with respect to the total amount of the water-absorbing resin particles and the fibrous materials may be, for example, 100% by mass or less, 95% by mass or less, 90% by mass or less, 80% by mass or less, 70% by mass or less, 60% by mass or less, or 50% by mass or less. That is, the proportion of the water-absorbing resin particles with respect to the total amount of the water-absorbing resin particles and the fibrous materials may be, for example, 2% to 100% by mass, 50% to 100% by mass, 60% to 100% by mass, or 70% to 100% by mass, and is preferably 80% to 100% by mass. Since the absorbent article according to the present embodiment comrprises the water-absorbing resin particles having a high release restoration rate, the permeation rate of the absorbent article can be maintained at a high rate even when the usage proportion of the fibrous material in the absorbent core is reduced.

The content of the water-absorbing resin particles in the absorbent is preferably 50 to 80 $g/m^2$, and preferably 60 to 70 $g/m^2$.

Examples of the material for the liquid-permeable sheet include non-woven fabrics formed of polyolefins such as polyethylene and polypropylene, polyesters, polyamides, or the like, and porous synthetic resin films. Examples of the material for the liquid-impermeable sheet include synthetic resin films formed of polyethylene, polypropylene, ethylene vinyl acetate, polyvinyl chloride, or the like; films formed of composites of these synthetic resins and non-woven fabrics; and films formed of composites of these synthetic resins with woven fabrics. These liquid-impermeable sheets may also be endowed with vapor-transmitting properties.

The absorbent and the absorbent article may further comprise components such as amorphous silica, deodorants, antibacterial agents, and fragrances.

A thickness of the absorbent article according to the present embodiment may be, for example, 2.0 mm or less, preferably 1.8 mm or less, more preferably 1.5 mm or less, and still more preferably 0.8 mm or less. The thickness of the absorbent article may be, for example, 0.4 mm or more or 1.0 mm or more. Since the absorbent article according to the present embodiment comprises the water-absorbing resin particles having excellent water absorption performance and release restoration rate, the absorbent article exhibits sufficient water absorption performance and permeation rate even though the thickness thereof is thin.

A thickness of the absorbent according to the present embodiment may be, for example, 1.2 mm or less, preferably 1.0 mm or less, more preferably 0.8 mm or less, and still more preferably 0.6 mm or less. The thickness of the absorbent may be, for example, 0.1 mm or more or 0.6 mm or more. Since the absorbent according to the present embodiment comprises the water-absorbing resin particles having excellent water absorption performance and release restoration rate, the absorbent exhibits sufficient absorption performance and permeation rate even in a case where the thickness thereof is thin.

A permeation rate of physiological saline of the absorbent article according to the present embodiment is preferably 15 seconds or less, more preferably 12 seconds or less, still more preferably 10 seconds or less, and further still more preferably 9 seconds or less in a measuring method described in Examples which will be described later.

The absorbent article according to the present embodiment can be applied to, for example, sanitary goods such as paper diapers, incontinence pads, and sanitary napkins, and industrial materials such as dew condensation-preventing agents, freshness-retaining agents, and drip sheets that absorb the liquid from foods.

EXAMPLES

Hereinafter, the present invention will be described in more detail with reference to Examples, but the present invention is not limited to these Examples.

[Example 1]

A cylindrical round-bottomed separable flask including sidewall baffles at four locations (baffle width: 7 mm) with an inner diameter of 110 mm and a capacity of 2 L, which was equipped with a reflux condenser, a dropping funnel, a nitrogen gas introduction tube, and a stirrer, was prepared. As the stirrer, stirring blades having four inclined paddle blades (surface-treated with a fluororesin) with a blade diameter of 50 mm in two stages were used. 660 ml of n-heptane as a petroleum-based hydrocarbon dispersion medium was put into the flask, 0.984 g of sorbitan monolaurate as a surfactant (Nonion LP-20R, HLB value of 8.6, manufactured by NOF Corporation) was added thereto, and the mixture was heated to 50°C at a rotational speed of the stirrer of 300 rpm. Sorbitan monolaurate was dissolved in n-heptane by heating, and then an internal temperature was lowered to 40°C.

92 g (1.02 moles) of an 80% by mass aqueous acrylic acid solution was put into an Erlenmeyer flask having a capacity of 500 ml, and 146 g of a 21% by mass aqueous sodium hydroxide solution was added dropwise thereto under ice-cooling of the aqueous acrylic acid solution from the outside to perform neutralization on acrylic acid. Next, 0.101 g (0.374 mmol) of potassium persulfate as a radical polymerization initiator was added to a partially neutralized aqueous acrylic acid solution thus obtained and dissolved to prepare an aqueous monomer solution.

The aqueous monomer solution was added to the separable flask and the inside of the system was sufficiently replaced with nitrogen. Then, a rotational speed of the stirrer was set to 700 rpm, and the flask was immersed in a water bath at 70°C and maintained for 60 minutes.

A liquid prepared by dispersing 0.092 g of amorphous silica (Carplex #80, manufactured by Evonik Degussa Japan, Inc.) as a powdery inorganic flocculating agent in 100 g of n-heptane was prepared. Then, in the separable flask, a rotational speed of the stirrer was set to 1,000 rpm, and the above liquid was added to a polymerization solution containing the produced hydrous gel, n-heptane, and a surfactant, and mixed for 10 minutes. Then, the flask containing a reaction solution was immersed in an oil bath at 125°C, and 109 g of water was extracted out of the system while n-heptane was refluxed by azeotropic distillation with n-heptane and water. Then, 9.94 g (2.85 mmol) of a 5% by mass aqueous ethylene glycol diglycidyl ether solution as a post-crosslinking agent was added to the flask, and the mixture was maintained at an internal temperature of 80±2°C for 2 hours.

After that, water and n-heptane were evaporated and the residue was dried until almost no evaporation substance from the inside of the system was distilled off, then the flask was once removed from the oil bath and 13.8 g of water was sprayed with a spray at a flow rate of 0.3 g per second. Then, the state was maintained at 80°C for 30 minutes while nitrogen is blown into the system at a flow rate of 200 ml per minute to obtain a dried product. The dried product was passed through a sieve having a mesh size of 850 μm to obtain 90.5 g of water-absorbing resin particles. The obtained water-absorbing resin particles were in the form in which granular (fine uneven) particles were aggregated.

In the obtained water-absorbing resin particles, a proportion of the particles having a size of more than 250 μm and 850 μm or less was 96% by mass and a proportion of the particles having a size of 250 μm or less was 4% by mass, with respect to all the particles. A median particle diameter was 410 μm.

[Example 2]

90.3 g of water-absorbing resin particles were obtained in the same manner as in Example 1 except that the amount of water extracted out of the system by azeotropic distillation was changed to 107 g. The obtained water-absorbing resin particles were in the form in which granular (fine uneven) particles were aggregated. In the obtained water-absorbing resin particles, a proportion of the particles having a size of more than 250 μm and 850 μm or less was 94% by mass and a proportion of the particles having a size of 250 μm or less was 6% by mass, with respect to all the particles. A median particle diameter was 380 μm.

[Example 3]

90.4 g of water-absorbing resin particles were obtained in the same manner as in Example 1 except that the amount of water extracted out of the system by azeotropic distillation was changed to 117 g. The obtained water-absorbing resin particles were in the form in which granular (fine uneven) particles were aggregated. In the obtained water-absorbing resin particles, a proportion of the particles having a size of more than 250 μm and 850 μm or less was 94% by mass and a proportion of the particles having a size of 250 μm or less was 6% by mass, with respect to all the particles. A median particle diameter was 390 μm.

[Example 4]

90.5 g of water-absorbing resin particles were obtained in the same manner as in Example 1 except that the amount of water extracted out of the system by azeotropic distillation was changed to 112 g and 4.97 g (2.85 mmol) of a 10% by mass aqueous ethylene glycol diglycidyl ether solution was added instead of a 5% by mass aqueous ethylene glycol diglycidyl ether solution as a post-crosslinking agent. The obtained water-absorbing resin particles were in the form in which granular (fine uneven) particles were aggregated. In the obtained water-absorbing resin particles, a proportion of the particles having a size of more than 250 $\mu$m and 850 $\mu$m or less was 91% by mass and a proportion of the particles having a size of 250 $\mu$m or less was 9% by mass, with respect to all the particles. A median particle diameter was 360 $\mu$m.

[Comparative Example 1]

A cylindrical round-bottomed separable flask including sidewall baffles at four locations (baffle width: 7 mm) with an inner diameter of 110 mm and a capacity of 2 L, which was equipped with a reflux condenser, a dropping funnel, a nitrogen gas introduction tube, and a stirrer, was prepared. As the stirrer, stirring blades having four inclined paddle blades (surface-treated with a fluororesin) with a blade diameter of 50 mm in two stages were used. 660 ml of n-heptane as a petroleum-based hydrocarbon dispersion medium was put into the flask, 1.10 g of sorbitan monolaurate as a surfactant (Nonion LP-20R, HLB value of 8.6, manufactured by NOF Corporation) was added thereto, and the mixture was heated to 50°C at a rotational speed of the stirrer of 300 rpm. Sorbitan monolaurate was dissolved in n-heptane by heating, and then an internal temperature was lowered to 40°C.

92 g (1.02 moles) of an 80% by mass aqueous acrylic acid solution was put into an Erlenmeyer flask having a capacity of 500 ml, and 146.0 g of a 21% by mass aqueous sodium hydroxide solution was added dropwise thereto under ice-cooling of the aqueous acrylic acid solution from the outside to perform neutralization of acrylic acid. Next, 0.101 g (0.374 mmol) of potassium persulfate as a radical polymerization initiator was added to a partially neutralized aqueous acrylic acid solution thus obtained and dissolved to prepare an aqueous monomer solution.

The aqueous monomer solution was added to the separable flask and the inside of the system was sufficiently replaced with nitrogen. Then, a rotational speed of the stirrer was set to 700 rpm, and the flask was immersed in a water bath at 70°C and maintained for 60 minutes. 0.41 g (0.047 mmol) of a 2% by mass aqueous ethylene glycol diglycidyl ether solution as an intermediate-crosslinking agent was added to the flask and the mixture was maintained at 75°C for 30 minutes.

A liquid prepared by dispersing 0.092 g of amorphous silica (Carplex #80, manufactured by Evonik Degussa Japan, Inc.) as a powdery inorganic flocculating agent in 100 g of n-heptane was prepared. In the separable flask, a rotational speed of the stirrer was set to 1,000 rpm, and the above liquid was added to a polymerization solution containing a hydrous gel, n-heptane, and a surfactant, and mixed for 10 minutes. After that, a reaction solution was heated in an oil bath at 125°C, and 129 g of water was extracted out of the system while n-heptane was refluxed by azeotropic distillation with n-heptane and water. Then, 4.14 g (0.48 mmol) of a 2% by mass aqueous ethylene glycol diglycidyl ether solution as a post-crosslinking agent was added, and the mixture was maintained at 80°C for 2 hours.

After that, water and n-heptane were evaporated and the residue was dried while nitrogen is blown into the system at a flow rate of 200 ml per minute to obtain a dried product. The dried product was passed through a sieve having a mesh size of 850 $\mu$m to obtain 90.0 g of water-absorbing resin particles. The obtained water-absorbing resin particles were in the form in which granular (fine uneven) particles were aggregated.

In the obtained water-absorbing resin particles, a proportion of the particles having a size of more than 250 $\mu$m and 850 $\mu$m or less was 92% by mass and a proportion of the particles having a size of 250 $\mu$m or less was 8% by mass, with respect to all the particles. A median particle diameter was 350 $\mu$m.

[Comparative Example 2]

A cylindrical round-bottomed separable flask with an inner diameter of 110 mm and a capacity of 2 L, which was equipped with a reflux condenser, a dropping funnel, a nitrogen gas introduction tube, and a stirrer, was prepared. As the stirrer, stirring blades having four inclined paddle blades with a blade diameter of 50 mm in two stages were used. 300 g of n-heptane as a petroleum-based hydrocarbon dispersion medium was put into the flask, and 0.74 g of sucrose stearic acid ester (manufactured by Mitsubishi-Chemical Foods Corporation, HLB 3, RYOTO sugar ester S-370) as a surfactant and 0.74 g of a maleic anhydride-modified ethylene-propylene copolymer (manufactured by Mitsui Chemicals, Inc., HI-WAX 1105A) as a polymer dispersant were added thereto, and the mixture was heated to 80°C at a rotational speed of the stirrer of 300 rpm. The surfactant was dissolved by heating and then cooled to 50°C.

92 g (1.02 moles) of an 80% by mass aqueous acrylic acid solution was put into an Erlenmeyer flask having a capacity of 500 mL, and 102.2 g of a 30% by mass aqueous sodium hydroxide solution was added dropwise thereto under cooling of the aqueous acrylic acid solution from the outside to perform neutralization of acrylic acid. After the neutralization, 0.092 g of hydroxylethyl cellulose (manufactured by Sumitomo Seika Chemicals, Ltd., HEC AW-15F) as a thickener, 0.074 g (0.274 mmol) of potassium persulfate as a peroxide, 0.018 g (0.106 mmol) of ethylene glycol diglycidyl ether as an internal crosslinking agent, and 43.8 g of ion exchange water were added and dissolved to prepare an aqueous monomer solution.

The aqueous monomer solution was added to the separable flask and the inside of the system was sufficiently replaced with nitrogen. Then, a rotational speed of the stirrer was set to 500 rpm, and the flask was immersed in a water bath at 70°C and maintained for 60 minutes to obtain a first-stage polymerized slurry.

128.8 g (1.43 moles) of an 80% by mass aqueous acrylic acid solution was put into another Erlenmeyer flask having a capacity of 500 mL, and 143.1 g of a 30% by mass aqueous sodium hydroxide solution was added dropwise thereto under cooling of the aqueous acrylic acid solution from the outside to perform neutralization of acrylic acid. After the neutralization, 0.104 g (0.382 mmol) of potassium persulfate as a peroxide, 0.039 g (0.222 mmol) of ethylene glycol diglycidyl ether as an internal crosslinking agent, and 15.9 g of ion exchange water were added to the flask and dissolved to prepare a second-stage aqueous monomer solution.

After that, a rotational speed of the stirrer was set to 1,000 rpm, the inside of the separable flask system was cooled to 25°C, and then the total amount of the second-stage aqueous monomer solution was added to the first-stage polymerized slurry. After the addition, the inside of the system was sufficiently replaced with nitrogen, and the flask was again immersed in a water bath at 70°C and maintained for 30 minutes.

After that, a reaction solution in the flask was heated in an oil bath at 125°C, and 273 g of water was extracted out of the system while n-heptane was refluxed by azeotropic distillation with n-heptane and water. Then, 6.63 g (0.761 mmol) of a 2% by mass aqueous ethylene glycol diglycidyl ether solution as a post-crosslinking agent was added to the flask, and the mixture was maintained at 80°C for 2 hours. After that, water and n-heptane were evaporated and the residue was dried while nitrogen is blown into the system at a flow rate of 200 ml per minute to obtain a dried product. This dried product was passed through a sieve having a mesh size of 850 $\mu$m to obtain 231.2 g of water-absorbing resin in the form in which spherical particles were aggregated.

In the obtained water-absorbing resin particles, a mass proportion of the particles having a size of 150 to 850 $\mu$m was 94% by mass with respect to the total proportion, and a median particle diameter was 390 $\mu$m.

[Comparative Example 3]

195.36 g of 100% acrylic acid was added to a separable flask having a capacity of 2 L. 135.13 g of ion exchange water was added while stirring the inside of the separable flask, and 357.93 g of 30% sodium hydroxide was added dropwise under an ice bath. Then, 104.73 g of 100% acrylic acid was added to prepare a partially neutralized acrylic acid solution.

780 g of the partially neutralized acrylic acid solution, 43.78 g of a 2% polyethylene glycol diacrylate (average repeating unit of ethylene oxide: 9) aqueous solution as an internal crosslinking agent solution, and 180.7 g of ion exchange water were introduced into a plastic bottle having a capacity of 2 L and mixed to prepare an aqueous monomer solution. The dissolved oxygen in the solution was adjusted to 0.1 ppm or less by pouring the aqueous monomer solution into a stainless steel vat and blowing nitrogen into the vat. Subsequently, under a nitrogen atmosphere, the temperature of the aqueous monomer solution was adjusted to 18°C, and then, under stirring, 1.58 g of a 5% aqueous sodium persulfate solution, 1.58 g of a 5% 2,2'-azobis(2-amidinopropane) dihydrochloride aqueous solution, 1.50 g of a 0.5% L-ascorbic acid aqueous solution, and 1.70 g of a 0.35% hydrogen peroxide aqueous solution were added dropwise in this order. After the hydrogen peroxide aqueous solution was added dropwise, the stirring was stopped. Polymerization was initiated immediately after the stop of the stirring, and after 9 minutes, the temperature of the aqueous monomer solution reached a peak temperature of 86°C. Subsequently, the vat was immersed in a hot water bath at 80°C and maintained for 10 minutes to obtain a hydrous gel of a crosslinked polymer.

Subsequently, the obtained hydrous gel of the crosslinked polymer was cooled to 30°C. The water content of the hydrous gel of the crosslinked polymer after the cooling was 63%. The hydrous gel of the crosslinked polymer was roughly crushed with a double-arm kneader having a capacity of 1 L and then dried at 180°C for 30 minutes to obtain a dried material. After that, the dried material was crushed using a crusher (rotor beater mill) with a screen hole size of 1 mm. After the crushing, a crushed material of 850 $\mu$m or more and a crushed material of less than 150 $\mu$m were removed to obtain water-absorbing resin particles having a median particle diameter of 380 $\mu$m (a mass proportion of the particles having a size of 150 $\mu$m or more and less than 850 $\mu$m was 100% by mass with respect to the total proportion).

[Evaluation Test of Water-Absorbing Resin Particles]

The water-absorbing resin particles obtained in Examples and Comparative Examples described above were subjected to various tests shown below and evaluated. The results are shown in Table 1.

(1) Retention Capacity of Physiological Saline

A cotton bag (Men broadcloth No. 60, 100 mm in width×200 mm in length) in which 2.00 g of the water-absorbing resin particles had been weighed was placed in a beaker having a capacity of 500 mL. 500 g of a 0.9% by mass sodium chloride aqueous solution (physiological saline) was poured into the cotton bag having the water-absorbing resin particles therein at once so that a lump could not be formed, and the upper part of the cotton bag was closed with a rubber band and left to stand for 30 minutes so that the water-absorbing resin particles were swollen. The cotton bag after an elapse of 30 minutes was dehydrated for 1 minute using a dehydrator (manufactured by Kokusan Co., Ltd., product number: H-122) which had been set at a centrifugal force of 167 G, and the mass Wa (g) of the cotton bag containing the swollen gel after dehydration was measured. By performing the same operation without addition of the water-absorbing resin particles, the mass Wb (g) of the empty cotton bag upon wetting was measured, and a retention capacity of physiological saline was calculated by the following formula.

$$\text{Retention capacity of physiological saline (g/g)} = [Wa - Wb]/2.00$$

(2) Absorption Rate of Physiological Saline

An absorption rate of physiological saline was measured by a vortex method in a room adjusted to $25°C \pm 1°C$. Specifically, $50 \pm 0.1$ g of physiological saline that had been put into a beaker having a capacity of 100 ml was adjusted to a temperature of $25°C \pm 0.2°C$ in a thermostatic water bath and then stirred at 600 rpm with a magnetic stirrer bar (8 mm$\varphi \times 30$ mm, no ring included) to generate a vortex. $2.0 \pm 0.002$ g of the water-absorbing resin particles were added into the physiological saline at once, and a time (sec) until the vortex disappeared and the liquid surface became flat after the addition of the water-absorbing resin particles was measured and the result was taken as an absorption rate of physiological saline of the water-absorbing resin particles.

(3) Particle Size Distribution

50 g of the water-absorbing resin particles were used for particle size distribution measurement. JIS standard sieves were combined in the following order from the top: a sieve with a mesh size of 850 $\mu$m, a sieve with a mesh size of 500 $\mu$m, a sieve with a mesh size of 425 $\mu$m, a sieve with a mesh size of 300 $\mu$m, a sieve with a mesh size of 250 $\mu$m, a sieve with a mesh size of 180 $\mu$m, a sieve with a mesh size of 150 $\mu$m, and a receiving tray.

The water-absorbing resin particles were fed to the topmost sieve in the combination and shaken for 20 minutes using a Ro-Tap shaker to conduct classification. After the classification, the mass of the water-absorbing resin particles remaining on each sieve was calculated as a mass percentage with respect to the total amount to determine a particle size distribution. By integrating the values on the sieves in descending order of the particle diameters with regard to the particle size distribution, the relationship between the mesh size of the sieve and the integrated value of the mass percentage of the water-absorbing resin particles remaining on the sieve was plotted on a logarithmic probability paper. By connecting the plotted points on the probability paper with straight lines, the particle diameter corresponding to 50% by mass of the integrated mass percentage was taken as a median particle diameter.

A presence proportion of the water-absorbing resin particles having a particle diameter of more than 250 $\mu$m and 850 $\mu$m or less is a sum of the proportions of the water-absorbing resin particles remaining on the sieves having mesh sizes of 500 $\mu$m, 425 $\mu$m, 300 $\mu$m, and 250 $\mu$m, and similarly, a presence proportion of the water-absorbing resin particles having a particle diameter of 250 $\mu$m or less is a numerical value obtained by adding up all the proportions of the water-absorbing resin particles remaining on the receiving tray and the sieves having mesh sizes of 180 $\mu$m and 150 $\mu$m.

(4) Release restoration rate Test of Water-Absorbing Resin Particles

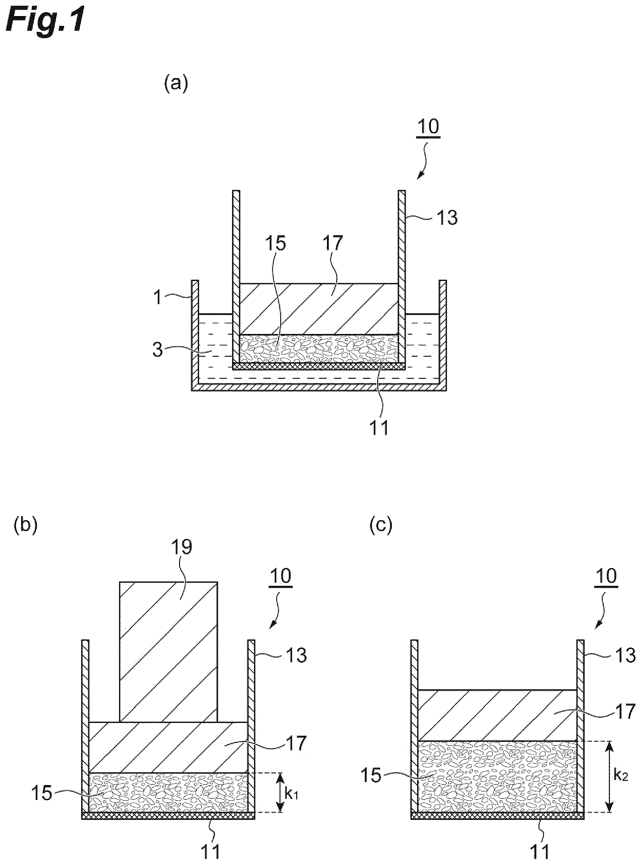

Fig. 1 is a schematic view illustrating a method of measuring the release restoration rate of the water-absorbing resin particles according to the present embodiment. 6.0 g of ion exchange water 3 was put into a Petri dish 1 (device 1: inner diameter of 48 mm, height of 15 mm). Next, 0.20 g of water-absorbing resin particles 15 were uniformly sprinkled inside a device 2 formed of an acrylic cylinder 10 (inner diameter of 25.4 mm, height of 35.6 mm, mass of 17.1 g) having a bottom surface to which a 100 mesh wire mesh 11 is adhered and graduated at intervals of 1 mm in a vertical height direction, and an acrylic piston 17 (device 3: inner diameter of 25.2 mm, mass of 5.6 g) was placed on the upper part of the cylinder. The inside of a side wall 13 of the cylinder 10 can be visually confirmed. At the same time when the device 2 was placed on the device 1, a stopwatch was started (t=0 minutes) to cause the water-absorbing resin particles 15 to absorb water. That is, the water-absorbing resin particles 15 are swollen by 30 times their own weight by the ion exchange water 3.

After 1 minute (t=1 minute), a metal weight 19 (device 4: outer diameter of 19.1 mm, mass of 59.8 g) was placed on the device 3. In this case, the pressure applied to the water-absorbing resin particles 15 is $1.3 \times 10^3$ Pa (0.19 Psi) when the weight 19 and the piston 17 are combined. After further 1 minute (t=2 minutes), the height under load $k_1$ mm (height from the bottom surface of the device 2 to the bottom surface of the device 3 (height from the top surface of the wire mesh 11 to the bottom surface of the piston 17. The same applies hereinbelow.)) was read, and at the same time, the device 4 was removed and the load was released. After further 1 minute (t=3 minutes), the restored height $k_2$ mm after the load release (height from the bottom surface of the device 2 to the bottom surface of the device 3) was read. Using the obtained numerical values of $k_1$ mm and $k_2$ mm, the release restoration rate was calculated from the following formula.

$$\text{Release restoration rate } (\%) = (k_2 - k_1)/k_1 \times 100$$

The water-absorbing resin particles of Examples 1 to 4 absorbed all the ion exchange water in the Petri dish within 1 minute from initiation of the water absorption.

[Table 1]

| | Absorption rate of physiological saline (sec) | Retention capacity of physiological saline (g/g) | Restored height after load release $k_2$ (mm) | Height under load $k_1$ (mm) | Release restoration rate $(k_2-k_1)/k_1 \times 100$ (%) |
|---|---|---|---|---|---|
| Example 1 | 5 | 18 | 22.8 | 19.2 | 18.8 |
| Example 2 | 4 | 18 | 23.4 | 19.8 | 18.2 |
| Example 3 | 4 | 29 | 16.4 | 14.5 | 13.1 |
| Example 4 | 5 | 35 | 13.6 | 12.7 | 7.3 |
| Comparative Example 1 | 4 | 34 | 11.6 | 11.6 | 0.3 |
| Comparative Example 2 | 43 | 31 | 15.0 | 14.4 | 4.0 |
| Comparative Example 3 | 70 | 40 | 12.6 | 12.3 | 2.3 |

[Evaluation of Absorbent Article]

(1) Preparation of Absorbent and Absorbent Article

Absorbents and absorbent articles were prepared using the water-absorbing resin particles obtained in Examples and Comparative Examples. 0.52 g of water-absorbing resin particles were uniformly sprinkled onto a tissue paper (16 cm$\times$5 cm) having a basis weight of 16 g/m$^2$ and then held with a tissue paper of the same size from above. Thus, a sheet-shaped absorbent was prepared. Further, an air-through type porous liquid-permeable sheet (thickness of 0.2 mm) made of polyethylene-polypropylene, which has the same size as the absorbent and has a basis weight of 22 g/m$^2$, was disposed on the upper surface of the absorbent, and a liquid-impermeable sheet (thickness of 0.1 mm) made of polyethylene, which has the same size and the same basis weight, was disposed on the lower surface of the absorbent to make the absorbent held therebetween. Thus, an absorbent article having a basis weight of water-absorbing resin particles of 65 g/m$^2$ was obtained.

(2) Thickness Measurement of Absorbent and Absorbent Article

Using a thickness measuring instrument (Ozaki mfg. Co., Ltd., model number: J-B), three locations of an upper end, a center, and a lower end (from the top, 2 cm was the upper end, 8 cm was the center, and 14 cm was the lower end) of the absorbent were measured along a longitudinal direction of the absorbent (16 cm$\times$5 cm). A measurement position in a width direction was set at the center. Measured values of the thickness were measured three times at each location and averaged, and the values at the upper end, the center, and the lower end were averaged to obtain the thickness of the absorbent. In addition, the same measurement was performed on the absorbent article.

(3) Permeation rate Evaluation Test of Absorbent Article

The absorbent article was placed on a horizontal laboratory table. A cylinder (mass of 60 g) equipped with a liquid inlet having an inner diameter of 2 cm was placed at the center of the absorbent article, 7 mL of physiological saline was introduced into the cylinder at once, and a time from the introduction of the liquid until the physiological saline in the cylinder disappeared was measured as a permeation time (sec) using a stopwatch. The results are shown in Table 2.

[Table 2]

| | Permeation rate evaluation test of absorbent article | | |
|---|---|---|---|
| | Permeation rate (sec) | Thickness of absorbent article (mm) | Thickness of absorbent (mm) |
| Example 1 | 6.9 | 0.6 | 0.3 |
| Example 2 | 7.1 | 0.6 | 0.3 |
| Example 3 | 8.2 | 0.5 | 0.2 |
| Example 4 | 8.6 | 0.6 | 0.3 |
| Comparative Example 1 | 11.8 | 0.5 | 0.2 |
| Comparative Example 2 | 15.0 | 0.6 | 0.3 |
| Comparative Example 3 | 15.4 | 0.5 | 0.2 |

## Reference Signs List

1...Petri dish, 3...ion exchange water, 10...cylinder, 11...wire mesh, 13...side wall, 15...water-absorbing resin particles, 17...piston, 19...weight, 20... absorbent article, 21...liquid-permeable sheet, 23...absorbent, 25...liquid-impermeable sheet, 27...absorbent core, 29...core wrap.

## Claims

1. An absorbent article comprising:

   an absorbent comprising water-absorbing resin particles,
   wherein the water-absorbing resin particles satisfy following requirements (a), (b), and (c),

   (a) a release restoration rate represented by Formula (1) is 5% or more:

$$(k_2 - k_1)/k_1 \times 100 \ (\%) \ ... \ (1)$$

   wherein in Formula (1), $k_1$ represents a height (mm) of the water-absorbing resin particles after a cylinder with an inner diameter of 25.4 mm, which has a mesh-like bottom onto which 0.2 g of the water-absorbing resin particles are sprinkled, is placed in a container having ion exchange water in an amount of 30 times a mass of the water-absorbing resin particles such that the water-absorbing resin particles absorb the ion exchange water and a load of $1.3 \times 10^3$ Pa is applied to the water-absorbing resin particles for 1 minute after 1 minute from initiation of the water absorption, and $k_2$ represents a height (mm) of the water-absorbing resin particles after 1 minute from release of the load applied when measuring $k_1$,
   (b) a water absorption rate measured by a vortex method is 5 seconds or less, and
   (c) a retention capacity of physiological saline is 15 to 39 g/g.

2. The absorbent article according to claim 1, wherein a content of the water-absorbing resin particles in the absorbent is 50 to 80 $g/m^2$.

3. The absorbent article according to claim 1 or 2, wherein the absorbent has a thickness of 1.2 mm or less.

4. The absorbent article according to any one of claims 1 to 3, wherein the absorbent article has a thickness of 2.0 mm or less.

5. The absorbent article according to any one of claims 1 to 4, wherein a permeation rate of physiological saline is 15 seconds or less.

*Fig.1*

(a)

(b)

(c)

*Fig.2*

## INTERNATIONAL SEARCH REPORT

<table>
<tr><td colspan="2"></td><td>International application No.<br>PCT/JP2019/013330</td></tr>
</table>

**A. CLASSIFICATION OF SUBJECT MATTER**
Int.Cl. A61F13/53(2006.01)i

According to International Patent Classification (IPC) or to both national classification and IPC

**B. FIELDS SEARCHED**

Minimum documentation searched (classification system followed by classification symbols)
Int.Cl. A61F13/15-13/84, A61L15/16-15/64

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2002-514934 A (THE PROCTER & GAMBLE COMPANY) 21 May 2002, page 9, lines 5-24, page 12, lines 24-29, page 22, line 17 to page 26, line 16, page 40, line 12 to page 42, line 17 & US 5843575 A, column 4, lines 24-51, column 6, lines 48-65, column 13, line 18 to column 15, line 66, column 24, line 44 to column 26, line 10 | 1-5 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 23 May 2019 (23.05.2019) | 04 May 2019 (04.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/013330 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 2006-57200 A (TORAY INDUSTRIES, INC.) 02 March 2006, paragraphs [0051]-[0054] (Family: none) | 1-5 |
| Y | JP 2011-32442 A (SAN-DIA POLYMER LTD.) 17 February 2011, paragraph [0044] (Family: none) | 1-5 |
| A | JP 9-3123 A (NIPPON SHOKUBAI CO., LTD.) 07 January 1997, entire text (Family: none) | 1-5 |

Form PCT/ISA/210 (continuation of second sheet) (January 2015)

**EP 3 777 802 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015154881 A